# EUROPEAN PATENT APPLICATION

(11) **EP 4 162 884 A1**
(43) Date of publication of application: **12.04.2023**
(21) Application number: 21818783.9
(22) Date of filing: 23.07.2021
(51) Int. Cl.: A61B 17/12

(54) **RELEASE DEVICE, SYSTEM, AND METHOD, AND THERAPEUTIC DEVICE**

(30) Priority: 05.06.2020 CN 202010506601
(71) Applicant: MicroPort NeuroTech (Shanghai) Co., Ltd., Shanghai 201318 (CN)
(72) Inventor: WANG, Xingzhao, Shanghai 201318 (CN); WEI, Lin, Shanghai 201318 (CN)
(74) Representative: Marks & Clerk LLP
(86) International application number: PCT/CN2021/108016
(87) International publication number: WO 2021/244671

(57) **Abstract**

A release device, system, and method, and a therapeutic device are provided. The release device (100) comprises a power supply assembly (110), a current detector (120), a first timer (130) and a control unit (140). The power consumption of the release circuit and the accumulated time duration over which the current of the release circuit are less than a predetermined current value are used as a determination index of whether the release process is successful or not. Thus, misjudgment caused by current fluctuation can be avoided, and determination accuracy is improved. At the same time, the release system uses a self-loop structure, and therefore no electric discharge needle or electrode need be inserted into the body of a patient during actual use of the device, reducing the suffering of the patient.

## Description

### TECHNICAL FIELD

The present invention relates to the technical field of medical instruments and, in particular, to a release device, system and method, and a therapeutic apparatus.

### BACKGROUND

Intracranial aneurysm is a common vascular disease that threatens middle-aged and elderly people. The aneurysm ruptures in patients due to high blood pressure. If no timely and effective therapeutic is given, it will be life-threatening. The traditional therapeutic method for intracranial aneurysm is mainly to clip the aneurysm by surgical operation, but it has the disadvantages of large trauma and high patient mortality.

In recent years, with the rapid development of interventional therapy technology, more and more patients with intracranial aneurysm choose interventional therapy. A commonly used interventional therapeutic method for intracranial aneurysm is to use a coil for embolization. The coil is connected to a push rod. The doctor pushes the push rod to deliver the coil to the diseased site where the intracranial aneurysm is located, and pushes the coil into the lumen of the aneurysm. After the coil is in place, the coil needs to be separated from the push rod, which requires a release process to be performed. The release process is carried out in the patient's body, and the doctor cannot observe the progress of the release. As a result, it is difficult for the doctor to determine the relase state. Therefore, it is eager to develop a release device capable of determining whether the coil has been completely released.

### SUMMARY OF THE INVENTION

An object of the present invention is to provide a release device, a relase system, a release method, and a therapeutic device, so as to improve the success rate in the release of the medical implant and improve the patient's use experience.

In order to achieve the above object, the present invention provides a release device, applied to a release system, the release device comprising a power supply assembly, a current detector, a first timer and a control unit; wherein,
the power supply assembly is configured to connect with a push rod and supply power to positive and negative electrodes of the push rod; when the power supply assembly supplies power to the positive and negative electrodes of the push rod, the release system forms a release circuit;
the current detector is configured to detect a current of the release circuit;
the first timer is configured to detect an accumulated time duration over which the current of the release circuit is less than a predetermined current value; and,
the control unit is in communication with the current detector and the first timer, and configured to acquire a power consumption of the release circuit and determine whether a release process performed by the release system is successful according to the power consumption and the accumulated time duration.

Optionally, the release device further comprises a second timer that is in communication with the control unit, and configured to detect a release time duration;
wherein the control unit is configured to calculate the power consumption according to the current of the release circuit and the release time duration.

Optionally, the control unit pre-stores a first predetermined timing and a first power consumption threshold, and the release device comprises a first release mode and a second release mode, wherein the control unit further pre-stores a first predetermined current value for the first release mode and a second predetermined current value for the second release mode, and the second predetermined current value is greater than the first predetermined current value;
wherein the control unit is further configured to acquire an initial power consumption that refers to a power consumption of the release circuit during a time period from a beginning of the release process to the first predetermined timing, and determine that the first release mode or the second release mode is to be excuted for the release process according to the initial power consumption and the first power consumption threshold.

Optionally, when the initial power consumption is less than the first power consumption threshold, the control unit is configured to execute the first release mode; the control unit further pre-stores a second predetermined timing and a third predetermined timing both for the first release mode, and the third predetermined timing is after the second predetermined timing;
during a time period from the beginning of the release process to the third predetermined timing, the first timer is configured to acquire a first accumulated time duration that refers to an accumulated time duration over which the current of the release circuit is less than the first predetermined current value in the a time period from the beginning of the release process to the third predetermined timing;
the control unit is configured to acquire a first power consumption that refers to a power consumption of the release circuit in a time period from the beginning of the release process to the second predetermined timing, and determine whether the release process is successful according to the first power consumption and the first accumulated time duration.

Optionally, the control unit pre-stores a second power consumption threshold and a first predetermined time duration, wherein the power consumption of the release circuit in the time period from beginning of the release process to the third predetermined timing is a second power consumption;
when the control unit determines whether the release process is successful, the control unit is configured to determine whether the first power consumption is less than the second power consumption threshold,
if so, acquire and store the second power consumption, and control the power supply assembly to stop supplying power to the release circuit to suspend the release process, and
if not, determine whether the first accumulated time duration is greater than or equal to the first predetermined time duration,
if not, acquire and store the second power consumption, and control the power supply assembly to stop supplying power to the release circuit to suspend the release process, and
if so, determine that the release process is successful.

Optionally, when the initial power consumption is greater than or equal to the first power consumption threshold, the control unit is configured to perform the release process in the second release mode;
the control unit pre-stores a fourth predetermined timing for the second release mode, and in a time period from the beginning of the release process to the fourth predetermined timing, the first timer is configured to acquire a second accumulated time duration that refers to an accumulated time duration over which the current of the release circuit is less than the second predetermined current value in the time period from the beginning of the release process to the fourth predetermined timing;
the control unit is configured to acquire a third power consumption that refers to the power consumption of the release circuit in the time period from the beginning of the release process to the fourth predetermined timing, and determine whether the release process is successful according to the third power consumption and the second accumulated time duration.

Optionally, the control unit pre-stores a third power consumption threshold and a second predetermined time duration;
when the control unit determines whether the release process is successful, the control unit is configured to determine whether the third power consumption is less than the third power consumption threshold,
if not, store the third power consumption, and control the power supply assembly to stop supplying power to the release circuit to suspend the release process, and
if so, determine whether the second accumulated time duration is greater than or equal to the second predetermined time duration,
if so, determine that the release process is successful, and
if not, store the third power consumption, and control the power supply assembly to stop supplying power to the release circuit to suspend the release process.

Optionally, the control unit further pre-stores a fifth predetermined timing and a fourth power consumption threshold, the fifth predetermined timing is after the fourth predetermined timing, and the fourth power consumption threshold is greater than the third power consumption threshold;
when the second accumulated time duration is greater than or equal to the second predetermined time duration, the control unit is configured to determine whether the third power consumption is greater than or equal to the fourth power consumption threshold, if so, determine that the release process is successful, and if not, control the power supply assembly to continue supplying power to the release circuit until the fifth predetermined timing is reached, and determine that the release process is successful at the fifth predetermined timing.

Optionally, the release device further comprises a third release mode, and the control unit further pre-stores a reference range of power consumption;
after the release process is suspended, the control device is further configured to determine whether the stored power consumption is within the reference range of power consumption, if yes, execute the third release mode, and if not, determine that the release process is terminated.

Optionally, the control unit pre-stores a third predetermined current value, a sixth predetermined timing and a fifth power consumption threshold;
in the third release mode, the control unit is configured to: acquire a fourth power consumption that refers to the power consumption of the release circuit in a time period from the beginning of the third release mode to the sixth predetermined timing; determine whether the fourth power consumption is greater than or equal to the fifth power consumption threshold; determine whether a comparison current is less than the third predetermined current value, the comparison current refers to the current of the release circuit at a timing for comparison, the timing for comparison refers to a timing when the power consumption of the release circuit in the third release mode is greater than the fifth power consumption threshold; determine whether the timing for comparison is before the sixth predetermined timing; determine whether the third accumulated time duration is greater than or equal to the third predetermined time duration, the third accumulated time duration is an accumulated time duration over which the current of the release circuit is less than the third predetermined current value in the time period from the beginning of the third release mode to the sixth predetermined timing, if the third accumulated time duration is greater than or equal to the third predetermined time duration, it is determined that the release process is successful.

Optionally, in the third release mode, the control unit is further configured to determine that the release process is terminated when the fourth power consumption is less than the fifth power consumption threshold, or when the comparison current is greater than the third predetermined current value.

Optionally, the release device further comprises a display element that is in communication with the control unit, and configured to display a release state of the release system.

Optionally, the power supply assembly comprises a constant current source and a current driver, wherein the constant current source and the current driver are both connected to the control unit, and the constant current source is configured to supply power to the release circuit through the current driver.

In order to achieve the above object, the present invention also provides a release system, comprising a push rod and the above release device, wherein the push rod is configured to connect with the power supply assembly of the release device, and the power supply assembly is configured to supply power to positive and negative electrodes of the push rod to form the release circuit.

Optionally, the power supply assembly has a positive electrode and a negative electrode, and the push rod comprises a first electrical conductor and a second electrical conductor, wherein the first electrical conductor is connected to the negative electrode of the power supply assembly, the second electrical conductor comprises a conductive area and a release area that are connected to each other, wherein the conductive area is connected to the positive electrode of the power supply assembly, and the release area is configured to connect with the first electrical conductor through an electrolyte, wherein the power supply assembly is configured to supply power to the push rod to break the release area.

In order to achieve the above object, the present invention also provides a therapeutic device, comprising a medical implant and the above release system, the medical implant is configurd to connect with the release system, and can be release from the release system in a predetermined condition.

In order to achieve the above object, the present invention also provides a release method, comprising steps of:
acquiring the power consumption of a release circuit;
acquiring an accumulated time duration over which a current of the release circuit is less than a predetermined current value; and
determining whether a release process performed by a release system is successful according to the power consumption and the accumulated time duration.

Optionally, the release process performed by the release system is determined to be successful when the power consumption reaches a predetermined power consumption threshold, and when the accumulated time duration reaches a predetermined time duration.

Optionally, the release method further comprises:
determining in advance whether the power consumption reaches the predetermined power consumption threshold, and if so, determining whether the accumulated time duration reaches the predetermined time duration.

Optionally, the predetermined current value is set according to a detection result of the present power consumption of the release circuit;
when the power consumption is less than the first power consumption threshold, a first predetermined current value is set;
when the power consumption is greater than or equal to the first power consumption threshold, a second predetermined current value is set;
wherein the first predetermined current value is less than the second predetermined current value.

Optionally, the release method further comprises a first release mode and a second release mode;
wherein the release method comprises:
Step S 1: determining whether the first release mode or the second release mode is to be selected for the release process to be performed for a first time according to whether the present power consumption of the release circuit reaches the first power consumption threshold;
Step S2: performing the release process for the first time, and determining that the release process performed for the first time is successful and the release process is terminated when the power consumption reaches a predetermined power consumption threshold and when the accumulated time duration reaches a predetermined time duration.

Optionally, the release method further comprises a third release mode;
when the release process is determined to be not successful in Step S2, the release method further comprises:
Step S3: determining whether the third release mode is to be excuted according to whether the power consumption of the release circuit reaches a preset range; if not, it is determinated that the release process is terminated, and if so, it is directed to Step S4;
Step S4: executing the third release mode to perform the release process for a second time; and determining that the release process performed for the second time is successful and the release process is terminated when the power consumption reaches the predetermined power consumption threshold and when the accumulated time duration reaches the predetermined time duration.

Optionally, in Step S1, the first release mode or the second release mode is selected for the release process to be performed for the first time according to:
Step S11: acquiring an initial power consumption that refers to a power consumption of the release circuit during a time period from a beginning of the release process to a first predetermined timing;
Step S12: determining whether the initial power consumption is less than the first power consumption threshold; if so, it is determined that the first release mode is to be selected for the release process; if not, it is detertmined that the second release mode is to be selected for the release process.

Optionally, the first predetermined current value, a second predetermined timing, a third predetermined timing, a second power consumption threshold and a first predetermined time duration are pre-stored for the first release mode, wherein the third predetermined timing is after the second predetermined timing;
wherein the first release mode is excuted through:
Step S21: acquiring a first power consumption that refers to the power consumption of the release circuit during a time period from the beginning of the release process to the second predetermined timing;
Step S22: determining whether the first power consumption is greater than or equal to the second power consumption threshold; if so, it is directed to Step S23 and Step S24; if not, it is directed to Step S25;
Step S23: acquiring a first accumulated time duration that refers to the accumulated time duration over which the current of the release circuit is less than the first predetermined current value during a time period from the beginning of release process to the third predetermined timing;
Step S24: determining whether the first accumulated time duration is greater than or equal to the first predetermined time duration; if so, it is determined that the release process is successful.

Optionally, when the release process is determined to be not successful in Step S24, the release method further comprises:
Step S25: acquiring and storing a second power consumption that refers to the power consumption of the release circuit in the time period from the beginning of the release process to the third predetermined timing, and stopping supplying power to the release circuit to suspend the release process.

Optionally, the second predetermined current value, a fourth predetermined timing, a fifth predetermined timing, a third power consumption threshold, a fourth power consumption threshold, and a second predetermined time duration are pre-stored for the second release mode, wherein the fifth predetermined timing is after the fourth predetermined timing, and the fourth power consumption threshold is greater than the third power consumption threshold;
the second release mode is excuted through:
Step S31: acquiring a third power consumption that refers to the power consumption of the release circuit in a time period from the beginning of the release process to the fourth predetermined timing;
Step S32: determining whether the third power consumption is greater than or equal to the third power consumption threshold; if so, it is directed to Step S33 and Step S34, and if not, it is directed to Step S37;
Step S33: acquiring a second accumulated time duration that refers to a time duration over which the current of the release circuit is less than the second predetermined current value during the time period from the beginning of release process to the fourth predetermined timing;
Step S34: determining whether the second accumulated time duration is greater than or equal to the second predetermined time duration; if so, it is directed to Step S35, and if not, it is directed to Step S37;
Step S35: determining whether the third power comsuption is greater than or equal to the fourth power consumption threshold; if so, it is determined that the release process is successful;
Step S37: storing the third power consumption, and stopping supplying power to the release circuit to suspend the release process.

Optionally, when it is determined that the release process is not successful in Step S35, the release method further comprises:
Step S36: supplying power to the release circuit until the fifth predetermined timing is reached, and determining that the release process is successful at the fifth predetermined timing.

Optionally, it is to be determined whether the stored power consumption is within a reference range of power consumption; if not, it is determined that the release process is terminated, and if so, it is determined that the third release mode is to be excuted.

Optionally, a third predetermined current value, a sixth predetermined timing, a fifth power consumption threshold and a third predetermined time duration are pre-stored for the third release mode;
the third release mode is excuted through:
Step S41: acquiring a fourth power consumption that refers to the power consumption of the release circuit in a time period from a beginning of the third release mode to the sixth predetermined timing;
Step S42: determining whether the fourth power consumption is greater than or equal to the fifth power consumption threshold; if not, it is determined that the first release mode is terminated.

Optionally, when it is determined that the fourth power consumption is greater than or equal to the fifth power consumption threshold in Step S42, it is directed to Step S43 and Step S44.
Step S43: acquiring a comparison current that refers to the current of the release circuit at the timing for comparison; the timing for comparison is a timing at which the power consumption of the release circuit in the third release mode is greater than the fifth power consumption threshold;
Step S44: determining whether the comparison current is less than the third predetermined current value; if so, it is directed to Step S45, and if not, it is directed to Step S46;
Step S45: determining whether the the timing for comparison is prior to the sixth predetermined timing; if so, it is directed to Step S46, and if not, it is determined that the second release mode is terminated;
Step S46: acquiring a third accumulated time duration that refers to a time duration over which the current of the release circuit is less than the third predetermined current value during the time period from the beginning of the third release mode to the sixth predetermined timing;
Step S47: determining whether the third accumulated time duration is greater than or equal to the third predetermined time duration; if so, it is determined that the release process is successful, and if not, it is directed to Step S43.

Compared with the prior art, the release device, system and method, and therapeutic device of the present invention have the following advantages:
The release device is applied to a release system, and includes a power supply assembly, a current detector, a first timer and a control unit. The power supply assembly is configured to connect with a push rod, and supply power to the positive and negative electrodes of the push rod. When the power supply assembly supplies power to the positive and negative electrodes of the push rod, the release system forms a release circuit. The current detector is configured to detect the current of the release circuit. The first timer is configured to detect the accumulated time duration over which the current is smaller than a predetermined current value. The control unit is in communication with the current detector and the first timer, and is configured to acquire the power consumption of the release circuit, and determine whether the release process of the release system is successful according to the power consumption and the accumulated time duration. The power consumption of the release circuit and the accumulated time duration over which the current of the release circuit are less than a predetermined current value are used for determining whether the release process is successful, which effectively reduces the misjudgment caused by the unstable current in the release circuit and improves the judgment accuracy.

The release device includes a first release mode and a second release mode, and an appropriate release mode and judgment criterion are selected according to the actual situation, which further improves the accuracy of the operator's judgment on the release state.

The release device further includes a third release mode. After the process release performed for the first time in the first release mode or the second release mode fails, if the power consumption of the release circuit satisfies a predetermined condition, the release device can automatically activate the third release mode to perform the release process for the second time. This improves the success rate of release, and improve the patient's experience of use.

### BRIEF DESCRIPTION OF THE DRAWINGS

Fig. 1 is a schematic structural diagram of a therapeutic device according to an embodiment of the present invention;
Fig. 2 is an overall frame diagram of a release device according to an embodiment of the present invention;
Fig. 3 is a flow chart showing the general working process of a release device according to an embodiment of the present invention;
Fig. 4 is a flow chart showing the working process of a release device in a first release mode according to an embodiment of the present invention;
Fig. 5 is a flow chart showing the working process a release device in a second release mode of according to an embodiment of the present invention;
Fig. 6 is a flow chart showing the working process of a third release mode of a release device according to an embodiment of the present invention.

### [List of Reference Numerals]:

100: release device;
110: power supply assembly;
111: constant current source, 112: current driver;
120: current detector;
130: first timer;
140: control unit, 141: storage module;
150: second timer;
160: display element;
200: push rod;
210: first electrical conductor;
220: second electrical conductor;
221: conductive area, 222: release area;
230: insulation;
300: medical implant.

### DETAILED DESCRIPTION

Objectives, advantages and features of the present invention will become more apparent upon reading the following more detailed description of the present invention, which is set forth by way of particular embodiments with reference to the accompanying drawings. Note that the figures are provided in a very simplified form not necessarily drawn to exact scale and for the only purpose of facilitating easy and clear despription of the embodiments.

As used herein, the singular forms "a", "an" and "the" include plural referents, unless the context clearly dictates otherwise. As used herein and in the appended claims, the term "or" is generally employed in the sense of "and/or", "plurality" of "two or more", and "several" of "one or more", unless the context clearly dictates otherwise. As used herein, unless otherwise expressly specified and defined, the terms "mounting", "coupling" and "connection" should be interpreted in a broad sense. For example, a connection may be a fixed, detachable or integral connection, or a mechanical or electrical connection, or a direct or indirect connection with one or more intervening media, or an internal communication or interaction between two components. Those of ordinary skill in the art can understand the specific meanings of the above-mentioned terms herein, depending on their context. The same or similar reference numerals in the drawings represent the same or similar parts.

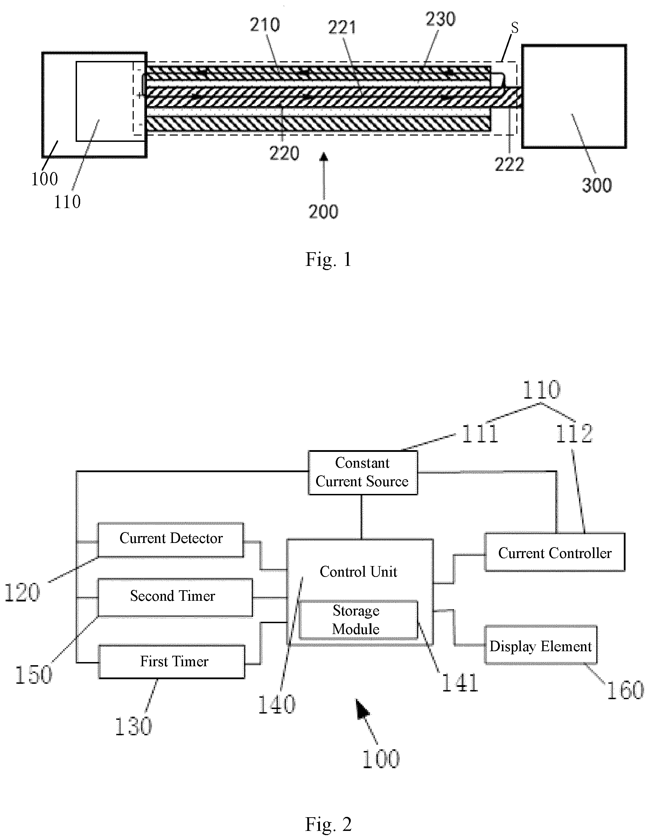

An object of the present invention is to provide a release device, which is applied to a release system that is configured to connect with a medical implant and to send the medical implant to a predetermined position in a patient's body. The medical implant includes, but is not limited to, coils. As shown in Fig. 1, the release system includes a release device 100 and a push rod 200. The push rod 200 includes a release area 222 that is configured to connect with the medical implant. The push rod 200 is configured to form a release circuit S together with the release device 100. In actual use, the operator first sends the medical implant 300 to a predetermined position in the patient's body through the push rod 200, then connects the push rod 200 and the release device 100, and then controls the release device 100 to supply electrical power to the push rod 200, so as to break the release area 222 of the push rod 200. As a result, the release of the medical implant 300 is complete.

Fig. 2 is an overall frame diagram of the release device 100 including a power supply assembly 110, a current detector 120, a first timer 130 and a control unit 140. The power supply assembly 110 is configured to connect with a push rod 200 and supply power to the positive and negative electrodes of the push rod 200. When the power supply assembly 110 supplies power to the positive and negative electrodes of the push rod 200, the release system forms a release circuit S. Further, the power supply assembly 110 can also supply power to the current detector 120, the first timer 130 and the control unit 140. The current detector 120 is in communication with the control unit 140 and configured to detect the current of the release circuit S. The first timer 130 is in communication with the control unit 140 and configured to detect the accumulated time duration over which the current of the release circuit S is less than the predetermined current. The control unit 140 is configured to acquire the power consumption of the release circuit S, and determine whether a release process performed by the release system is successful according to the power consumption and the accumulated time duration.

Generally, as the release process progresses, the current of the release circuit S changes in real time. The release device 100 according to the embodiment of the present invention is configured to calculate the power consumption of the release circuit S based on the current of the release circuit S. The first timer 130 is configured to detect the accumulated time duration over which the current of the release circuit S is less than a predetermined current. The power consumption and the accumulated time duration are used together for determining the release state of the release system, so that the possibility of misjudgment due to the instable current can be reduced. Specifically, when the power consumption reaches the predetermined power consumption threshold, and the accumulated time duration reaches the predetermined time duration, it can be determined that the release process of the release system is successful. In this embodiment, the control unit 140 is implemented as a programmable control unit known to those skilled in the art, such as a PLC controller, a computer, and the like.

Optionally, the release device 100 further includes a second timer 150 that is in communication with the control unit 140, and configured to detect the release time duration of the release circuit S (that is, the time duration over which the power supply assembly outputs current to the release circuit S). The control unit 140 is configured to calculate the power consumption according to the current of the release circuit S and the release time duration, which for example is an integral of the current of the release circuit S with respect to the release time duration.

Optionally, the release device 100 may further include a display element 160 that is in communication with the control unit 140, and configured to display the release state of the release system, for example display that the release process is successful, the release process is suspended, the release process is terminated, etc. The display element 160 may be a buzzer alarm, a display screen, an LED light and other components.

Optionally, the power supply assembly 110 preferably includes a constant current source 111 and a current driver 112. The constant current source 111 may be a DC constant current source, and the constant current source 111 is configured to supply power directly to the current detector 120, the first timer 130, the control unit 140, the second timer, and the display element 160. The control unit 140 controls the supply of a constant direct current to the release circuit S by controlling power-on and power-off of the current driver 112. The constant direct current may be in the range of 1.2-3.0 mA, preferably 1.8 mA.

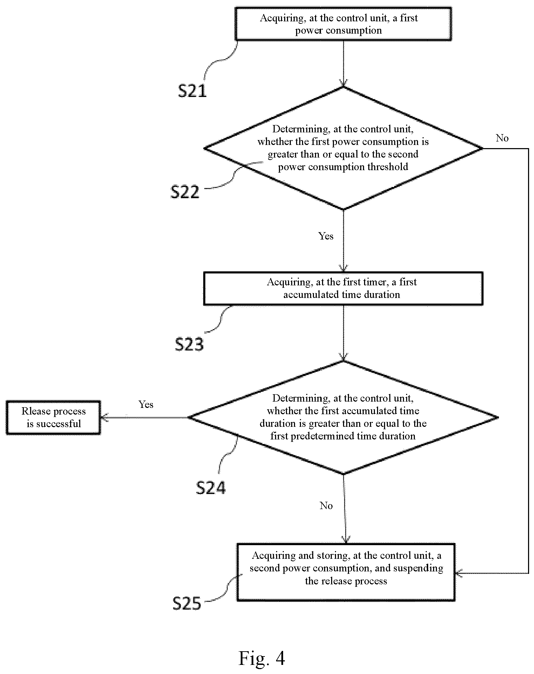

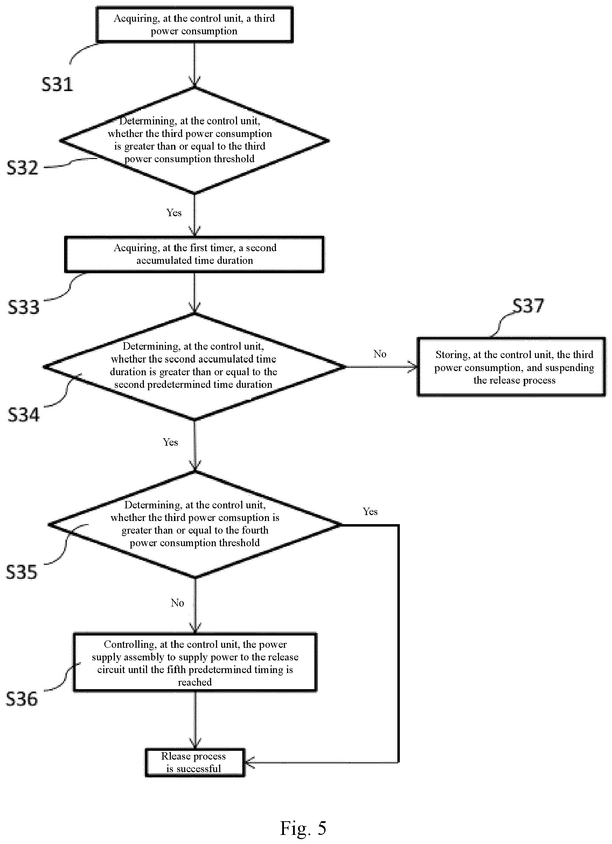

During the release of the implant, the releasing device 100 performs a release process at least once. Specifically, the release device 100 includes a first release mode and a second release mode. The control unit 140 may select the first release mode or the second release mode for performing the releae process for the first time according to the actual situation of the release system 10. Specifically, it is determined whether the first release mode or the second release mode is to be selected to perform the release process for the first time according to the determination whether the power consumption of the release circuit S reaches a first power consumption threshold. If the power consumption of the release circuit S is less than the first power consumption threshold, the first release mode is selected. If the power consumption of the current release circuit S is greater than or equal to the first power consumption threshold, the second release mode is selected. Please see Fig. 4 for the first release mode, and see Fig. 5 for the second release mode. The control unit 140 may further include a storage module 141. The storage module 141 pre-stores a first predetermined current value for the first release mode, and a second predetermined current value for the second release mode. The second predetermined current value is greater than the first predetermined current value.

The release device 100 further includes a third release mode configured to perform the release process for the second time.

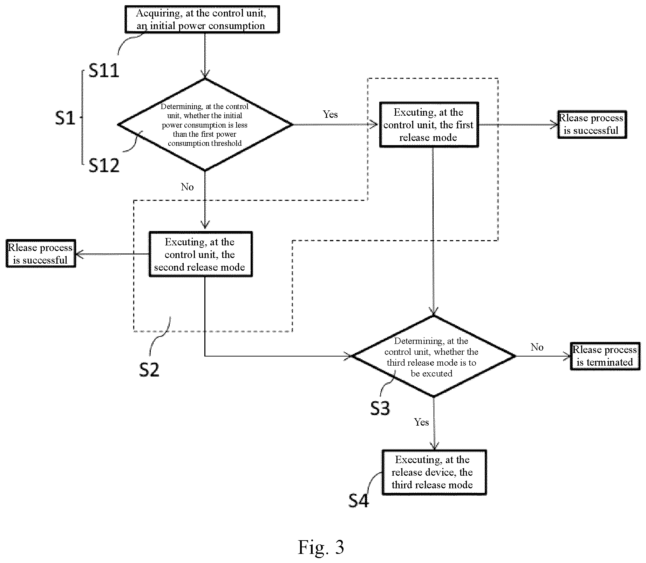

Fig. 3 is a flow chart showing the general working process of a release device according to an exemplary embodiment. Before the release process begins, the storage module 141 pre-stores parameters such as the first power consumption threshold, the first predetermined timing, and the reference range of power consumption. The first predetermined timing may be 0.5-1.5 s, for example, 1 s, since the release process begins. The first power consumption threshold may be within a range of 0.2-2.2 mAs, for example, 1 mAs. The reference range of power consumption may be in a range of 1-24 mAs, and preferably 2-16 mAs.

As shown in Fig. 3, the working process of the release system for releasing the implant is as follows:
Step S 1: determining, at the control unit, whether the first release mode or the second release mode is to be selected for the release process to be performed for the first time;
Step S2: controlling the release device to perform the release process for the first time, and determining whether the release process performed for the first time is successful, if so, the release process is terminated, if not, it is directed to Step S3;
Step S3: determining, at the control unit, whether the third release mode is to be excuted, if not, the release process is terminated, if so, it is directed to Step S4;
Step S4: selecting, at the release device, the third release mode to perform the release process for the second time.

The process of Step S1 is specifically as follows:
Step S11: acquiring, at the control unit, an initial power consumption, which refers to the power consumption of the release circuit during the time period from the beginning of the release process to the first predetermined timing.
Step S12: determining, at the control unit, whether the initial power consumption is less than the first power consumption threshold; if so, it is determined to select the first release mode for the release process to be performed for the first time; if not, it is detertmined to select the second release mode to perform the release process for the first time.

The control unit 140 of the release device can detect and distinguish the current environment of the release circuit S, and select different release modes (i.e., the first release mode or the second release mode) according to the current environment. This effectively avoids the "one-size-fits-all" standard for detection of release state, improves the accuracy of judgment, and reduces misjudgment.

The specific processes of the first release mode, the second release mode and the third release mode will be described in detail below with reference to Figs. 4 to 6.

Fig. 4 is a flow chart of the first release mode according to an exemplary embodiment. In the first release mode, the storage module pre-stores a first predetermined current value that is an index for determining whether the release area is about to break. In the first release mode, the control unit further pre-stores a second predetermined timing, a third predetermined timing, a second power consumption threshold and a first predetermined time duration. According to specific conditions, the first predetermined current value may be in a range of 0.1-0.8 mA, such as 0.2 mA; the second predetermined timing may be in a range of 5-8 s, such as 7.5 s, since the beginning of the release process; the third predetermined timing may be in a range of 8-13 s, such as 10 s, since the beginning of the release process; the second power consumption threshold may be in a range of 2-5.4 mAs, such as 3 mAs; the first predetermined time duration may be in a range of 0.5-1.8 s, such as 1 s.

As shown in Fig. 4, the process of the first release mode is as follows:
Step S21: acquiring, at the control unit, a first power consumption, which refers to the power consumption of the release circuit during the time period from the beginning of the release process to the second predetermined timing;
Step S22: determining, at the control unit, whether the first power consumption is greater than or equal to the second power consumption threshold. If so, it is directed to Step S23 and Step S24; if not, it is directed to Step S25;
Step S23: acquiring, at the first timer, a first accumulated time duration, which refers to the accumulated time duration over which the current of the release circuit is less than the first predetermined current value during the time period from the beginning of release process to the third predetermined timing;
Step S24: determining, at the control unit, whether the first accumulated time duration is greater than or equal to the first predetermined time duration; if so, it is determined that the release process is successful; if not, it is directed to Step S25;
Step S25: acquiring and storing, at the control unit, a second power consumption, which refers to the power consumption of the release circuit during the time period from the beginning of the release process to the third predetermined timing, and controlling the power supply assembly to stop supplying power to the release circuit to suspend the release process.

Preferably, the control unit is configured to control the power supply assembly to stop supplying power to the release circuit at the third predetermined timing. That is, the release process is preferably to be suspended at the third predetermined timing.

In the first release mode, the current supplied from the power supply assembly to the release circuit is small, so the power consumption of the release circuit increases slowly. The control unit determines whether the first power consumption reaches the second power consumption threshold at a timing between the second predetermined timing and the thrid predetermined timing. In this way, the workload of the control unit can be reduced.

Fig. 5 is a flow chart of the second release mode according to an exemplary embodiment. In the second release mode, the control unit pre-stores a second predetermined current value, a fourth predetermined timing, a fifth predetermined timing, a third power consumption threshold, a fourth power consumption threshold and a second predetermined time duration. The second predetermined current value is greater than the first predetermined current value, and may be in a range of 0.2-1.8 mA, such as 0.6 mA. The fourth predetermined timing may be in a range of 7.5-9.5 s, such as 8.5 s, since the beginning of the release process. The fifth predetermined timing may be in a range of 8-13 s, such as 10 s, since the beginning of the release process. The third power consumption threshold may be in a range of 2.2-5.4 mAs, such as 3 mAs. The fourth power consumption is greater than the third power consumption threshold, and may be in a range of 2.2-5.4 mAs, such as 3.4 mAs. The second predetermined time duration may be in a range of 0.5-2.4 s, such as 1 s.

As shown in Fig. 5, the process of the second release mode is as follows:
Step S31: acquiring, at the control unit, a third power consumption, which refers to the power consumption of the release circuit during the time period from the beginning of the release process to the fourth predetermined timing;
Step S32: determining, at the control unit, whether the third power consumption is greater than or equal to the third power consumption threshold; if so, it is directed to Step S33 and Step S34, and if not, it is directed to Step S37;
Step S33: acquiring, at the first timer, a second accumulated time duration, which refers to the time duration over which the current of the release circuit is less than the second predetermined current value during the time period from the beginning of release process to the fourth predetermined timing;
Step S34: determining, at the control unit, whether the second accumulated time duration is greater than or equal to the second predetermined time duration; if so, it is directed to Step S35, and if not, it is directed to Step S37;
Step S35: determining, at the control unit, whether the third power comsuption is greater than or equal to the fourth power consumption threshold; if so, it is determined that the release process is successful; if not, it is directed to Step S36;
Step S36: controlling, at the control unit, the power supply assembly to supply power to the release circuit until the fifth predetermined timing is reached, and determining that the release process is successful at the fifth predetermined timing;
Step S37: storing, at the control unit, the third power consumption, and controlling the power supply assembly to stop supplying power to the release circuit to suspend the release process.

The control unit determines whether the third release mode to be excuted according to the second power consumption or the third power consumption. That is, if the release device selects the first release mode for the release process to be performed for the first time, the control unit determines whether the second power consumption is within the reference range of power consumption; if so, it is determined to execute the third release mode, if not, it is determined that release process is terminated. If the release device selects the second release mode for the release process to be performed for the first time, the control unit determines whether the third power consumption is within the reference range of power consumption; if so, it is determined to execute the third release mode, if not, it is determined that the release process is terminated.

The control unit is configured to determine whether the release process is to be performed for the second time according to the power consumption of the release circuit when the release process performed for the first time is suspended, which can reduce the unnecessary suffering of the patient. This is because if the power consumption of the release circuit is less than the minimum value of the reference range of power consumption when the release process performed for the first time is suspended, it means that the current of the release circuit is small, which may be due to poor release environment or short-circuiting of the release circuit; if the power consumption of the relase circuit is greater than the maximum value of the reference range of power consumption when release process performed for the first time is suspended, it means that the current of the release circuit is large, and a short circuit may have occurred. The existence of these problems will cause the release system to fail to complete the release process, thereby eliminating the need for a release process to be perfomed for the second time.

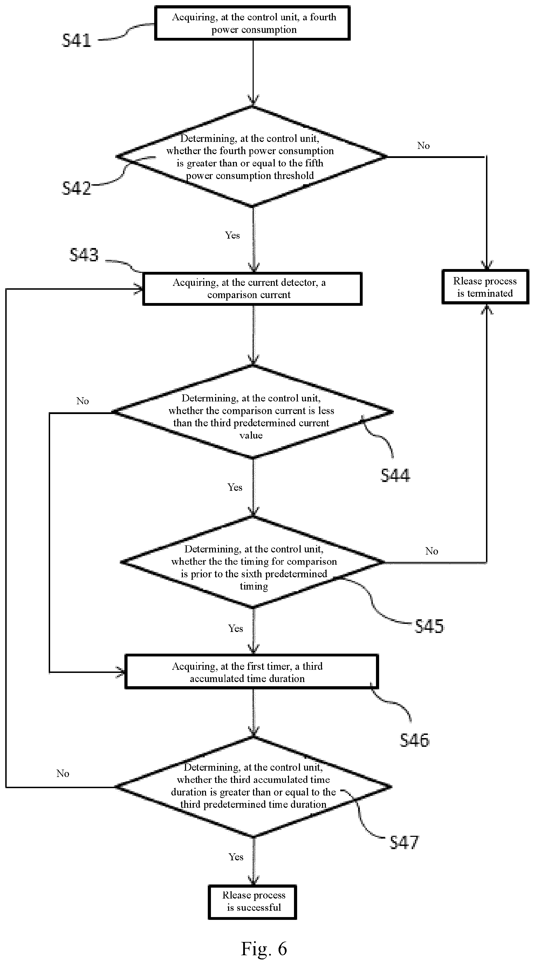

Fig. 6 is a flow chart of the third release mode according to an exemplary embodiment. In the third release mode, the control unit pre-stores a third predetermined current value, a sixth predetermined timing, a fifth power consumption threshold and a third predetermined time duration. The third predetermined current value may be in a range of 0.1-0.6 mA, such as 0.2 mA. The sixth predetermined timing may be in a range of 8-12 s, such as 10 s, since the beginning of the third predetermined mode. The fifth power consumption threshold may be in a range of 1.2-4.5 mAs, such as 2 mAs. The third predetermined time duration may be in a range of 0.2-2.4 s, such as 1 s.

Before executing the third release mode, preferably the control unit 140 clears the previously stored power consumption (i.e., the second power consumption or the third power consumption), so that the power consumption calculated by the control unit after the third relase mode is excuted is actually the power consumption of the release circuit in the third relase mode. As shown in Fig. 6, the process of the third release mode is as follows:
Step S41: acquiring, at the control unit, a fourth power consumption, which refers to the power consumption of the release circuit in the time period from the beginning of the third release mode to the sixth predetermined timing;
Step S42: determining, at the control unit, whether the fourth power consumption is greater than or equal to the fifth power consumption threshold; if so, it is directed to Step S43 and Step S44, and if not, it is determined that the release process is terminated;
Step S43: acquiring, at the current detector, a comparison current, which refers to the current of the release circuit at the timing for comparison; the timing for comparison refers to any timing at which the power consumption of the release circuit in the process of excution of the third release mode is greater than the fifth power consumption threshold;
Step S44: determining, at the control unit, whether the comparison current is less than the third predetermined current value; if so, it is directed to Step S45, and if not, it is directed to Step S46;
Step S45: determining, at the control unit, whether the the timing for comparison is prior to the sixth predetermined timing; if so, it is directed to Step S46, and if not, it is determined that the release process is terminated;
Step S46: acquiring, at the first timer, a third accumulated time duration, which refers to the time duration over which the current of the release circuit is less than the third predetermined current value in the time period from the beginning of the third release mode to the sixth predetermined timing;
Step S47: determining, at the control unit, whether the third accumulated time duration is greater than or equal to the third predetermined time duration; if so, it is determined that the release process is successful, and if not, it is directed to Step S43.

In Step S42, if the fourth power consumption is less than the fifth power consumption threshold, the control unit can determine that the first release mode is terminated, and further determine that the release process is terminated. In Step S45, if the timing for comparison is after the sixth predetermined timing, it means that the current of the release circuit has not decreased to the third predetermined current value before the sixth predetermined timing, and the control unit determines that the second release mode is terminated, and further determines that the release process is terminated.

In another embodiment, the order of Step S44 and Step S45 in Fig. 6 can also be interchanged. In this case, the process of the third release mode is as follows:
Step S41: acquiring, at the control unit, a fourth power consumption, which refers to the power consumption of the release circuit in the time period from the beginning of the third release mode to the sixth predetermined timing;
Step S42: determining, at the control unit, whether the fourth power consumption is greater than or equal to the fifth power consumption threshold; if so, it is directed to Step S43 and Step S45, and if not, it is determined that the release process is terminated;
Step S43: acquiring, at the current detector, a comparison current, which refers to the current of the release circuit at the timing for comparison; the timing for comparison refers to any timing at which the power consumption of the release circuit in the process of excution of the third release mode is greater than the fifth power consumption threshold;
Step S45: determining, at the control unit, whether the the timing for comparison is prior to the sixth predetermined timing; if so, it is directed to Step S44, and if not, it is determined that the release process is terminated;
Step S44: determining, at the control unit, whether the comparison current is less than the third predetermined current value; if so, it is directed to Step S46;
Step S46: acquiring, at the first timer, a third accumulated time duration, which refers to the time duration over which the current of the release circuit is less than the third predetermined current value in the time period from the beginning of the third release mode to the sixth predetermined timing;
Step S47: determining, at the control unit, whether the third accumulated time duration is greater than or equal to the third predetermined time duration; if so, it is determined that the release process is successful, and if not, it is directed to Step S43.

In addition, in the third release mode, the current output by the constant current source 111 to the release circuit may be in a range of 1.2-3.0 mA, for example, 1.8 mA.

Based on the aforementioned release device, an embodiment of the present invention further provides a release system. As shown in Fig. 1, the release system includes a release device 100 and a push rod 200. The push rod 200 is connected to the power supply assembly 110 of the release device 100, and the power supply assembly 110 is configured to supply power to the positive and negative electrodes of the push rod 200 to form a release circuit S.

In detail, the power supply assembly 110 has a positive electrode and a negative electrode. The push rod 200 includes a first electrical conductor 210 and a second electrical conductor 220. The proximal end of the first electrical conductor 210 is connected to the negative electrode of the power supply assembly 110. The second conductor 220 includes a conductive area 221 and a release area 222 that are connected to each other. The proximal end of the conductive area 221 is connected to the positive electrode of the power supply assembly 110. In this way, the proximal end of the first electrical conductor 210 is formed as the negative electrode of the push rod 200, and the proximal end of the conductive area 221 of the second electrical conductor 220 is formed as the positive electrode of the push rod 200. The release area 222 is disposed at the distal end of the conductive area 221, and is connected to the second end through an electrolyte such as body fluid to form the release circuit S. It can be understood that the "proximal end" and "distal end" mentioned here refer to relative orientations, relative positions and directions of elements relative to each other from the perspective of the doctor operating the medical instruments. Although the "proximal end" and the "distal end" are not restrictive, the "proximal end" generally refers to the end of the medical device that is close to the doctor during normal operation, while the "distal end" generally refers to the end that enters the patient's body first.

In an exemplary embodiment, the first electrical conductor 210 and the second electrical conductor 220 are arranged one sleeved over another. In detail, please continue to refer to Fig. 1, the first electrical conductor 210 has a columnar body and has an inner cavity axially extending therethrough. The conductive area 221 of the second electrical conductor 220 is in the inner cavity, the release area 222 extends to the outside of the inner cavity. An insulation 230 is further disposed between the conductive area 221 and the first electrical conductor 210.

Please continue to refer to Fig. 1, based on the aforementioned release system, an embodiment of the present invention further provides a therapeutic device. The therapeutic device includes a medical implant 300 and the aforementioned release system. The medical implant 300 is configued to connect to the release system, and can be released from the release system under predetermined conditions. More specifically, the medical implant 300 is connected to the release area 222 of the push rod 200. The medical implant 300 described here includes, but is not limited to, a coil.

When a doctor uses the therapeutic device for interventional treatment, such as embolization treatment for intracranial aneurysm, the medical implant 300 can be sent into the lumen where the intracranial aneurysm locates by the push rod 200, and then the release device 100, the push rod 200 and the body fluid together form a release circuit S, to which the power supply assembly 110 of the release device 100 supplies power so as to break the release area 222 to complete the relase of the medical implant 300. The medical implant 300 may be a coil or a stent. The therapeutic device provided in this embodiment adopts a self-loop structure, so that there is no need to insert conductive needles or place electrodes on the patient's body, thereby reducing the suffering of the patient.

Further, an embodiment of the present invention also provides a release method including the following steps:
acquiring the power consumption of the release circuit;
detecting the accumulated time duration over which the current of the release circuit is less than the predetermined current value; and
determining whether the release process performed by the release system is successful according to the power consumption and the accumulated time duration.

Whether the release process is successful can be determined according to whether the power consumption reaches a predetermined power consumption threshold and whether the accumulated time dration reaches the predetermined time duration; if both so, it is determined that the release process of the release system is successful. Preferably, it is to be determined in advance whether the power consumption reaches a predetermined power consumption threshold; if so, it is then to be determined whether the accumulated time duration has reached the predetermined time duration.

Preferably, the predetermined current value is set according to the detection result about the present power consumption of the release circuit. When the power consumption is less than the first power consumption threshold, the first predetermined current value is set. When the power consumption is greater than or equal to the first power consumption threshold, the second predetermined current value is set. The first predetermined current value is less than the second predetermined current value. This method distinguishes between high-current environments and low-current environments, and adopts different judgment standards and different judgment steps for different current environments, which is more flexible and accurate. In an embodiment, the power consumption of the release circuit is acquired, a first predetermined current value is set when the power consumption is less than a first power consumption threshold, and a second predetermined current value is set when the power consumption is greater than or equal to the first power consumption threshold. The first predetermined current value is less than the second predetermined current value. The release environments are distinguished into a low-current environment and a high-current environment. A first predetermined current value is set in the low-current environment. Whether the release process is successful can be determined according to whether the present power consumption of the release circuit reaches a predetermined power consumption threshold and whether the accumulated time dration over which the current of the release circuit is less than or equal to the first predetermined current value reaches the predetermined time duration; if both so, it is determined that the release process of the release system is successful. A second predetermined current value (greater than the first predetermined current value in the low-current environment) is set in the high-current environment. Whether the release process is successful can be determined according to whether the present power consumption of the release circuit reaches a predetermined power consumption threshold and whether the accumulated time dration over which the current of the release circuit is less than or equal to the second predetermined current value reaches the predetermined time duration; if both so, it is determined that the release process of the release system is successful.

In another embodiment, in the release method, the release process under a low-current environment and the release process under a high-current environment are respectively defined as a first release mode and a second release mode (the names of the two can also be interchanged). The release process under the first release mode or the second release mode is to be performed for the first time. The release method includes the following steps:
Step S1: determining whether the first release mode or the second release mode is to be selected for the release process to be performed for the first time according to whether the present power consumption of the release circuit reaches the first power consumption threshold;
Step S2: performing the release process for the first time, and determining that the release process performed for the first time is successful and the release process is terminated when the power consumption reaches a predetermined power consumption threshold and when the accumulated time duration reaches a predetermined time duration.

In another embodiment, referring to Fig. 3, when the release process in the first release mode or the second release mode is not successful, a third release mode is further included in the release method. If the release process is determined to be not successful in Step S2, in additional to the aforementioned steps, the relase method further includes:
Step S3: determining whether the third release mode is to be excuted according to whether the power consumption of the release circuit reaches a preset range; if not, it is determinated that the release process is terminated, and if so, it is directed to Step S4;
Step S4: executing the third release mode to perform the release process for the second time; and determining that the release process performed for the second time is successful and the release process is terminated when the power consumption reaches the predetermined power consumption threshold and when the accumulated time duration reaches the predetermined time duration.

If the release system fails to perform the release process for the first time and the release process is suspended, the control unit will store the power consumption of the release circuit at a predetermined timing, and determine whether the third release mode is to be excuted so that the release process is to be performed for the second time according to the power consumption of the release circuit at the predetermined timing.

In another specific embodiment, in Step S1, the first release mode or the second release mode is selected for the release process to be performed for the first time based on following steps:
Step S11: acquiring an initial power consumption, which refers to the power consumption of the release circuit in the time period from the beginning of the release process to the first predetermined timing;
Step S12: determining whether the initial power consumption is less than the first power consumption threshold; if so, it is determined to select the first release mode to perform the release process for the first time; if not, it is detertmined to select the second release mode to perform the release process for the first time.

In another embodiment, referring to Fig. 4, the process of the first release mode is as follows:
Step S21: acquiring a first power consumption, which refers to the power consumption of the release circuit in the time period from the beginning of the release process to the second predetermined timing;
Step S22: determining whether the first power consumption is greater than or equal to the second power consumption threshold. If so, it is directed to Step S23 and Step S24; if not, it is directed to Step S25;
Step S23: acquiring a first accumulated time duration, which refers to the accumulated time duration over which the current of the release circuit is less than the first predetermined current value in the time period from the beginning of release process to the third predetermined timing;
Step S24: determining whether the first accumulated time duration is greater than or equal to the first predetermined time duration; if so, it is determined that the release process is successful.

In this embodiment, the first predetermined current value, the second predetermined timing, the third predetermined timing, the second power consumption threshold and the first predetermined time duration are pre-stored for the first release mode. The third predetermined timing is after the second predetermined timing.

In another embodiment, when it is determined that the release process is not successful in Step S24, the release method further includes:
Step S25: acquiring and storing a second power consumption, which refers to the power consumption of the release circuit in the time period from the beginning of the release process to the third predetermined timing, and stopping supplying power to the release circuit to suspend the release process.

In one embodiment, referring to Fig. 5, the process of the second release mode is as follows:
Step S31: acquiring a third power consumption, which refers to the power consumption of the release circuit in the time period from the beginning of the release process to the fourth predetermined timing;
Step S32: determining whether the third power consumption is greater than or equal to the third power consumption threshold; if so, it is directed to Step S33 and Step S34, and if not, it is directed to Step S37;
Step S33: acquiring a second accumulated time duration, which refers to the time duration over which the current of the release circuit is less than the second predetermined current value in the time period from the beginning of release process to the fourth predetermined timing;
Step S34: determining whether the second accumulated time duration is greater than or equal to the second predetermined time duration; if so, it is directed to Step S35, and if not, it is directed to Step S37;
Step S3 5: determining whether the third power comsuption is greater than or equal to the fourth power consumption threshold; if so, it is determined that the release process is successful;
Step S37: storing the third power consumption, and stopping supplying power to the release circuit to suspend the release process.

In this embodiment, the second predetermined current value, the fourth predetermined timing, a fifth predetermined timing, the third power consumption threshold, the fourth power consumption threshold and the second predetermined time duration are pre-stored for the second release mode. The fifth predetermined timing is after the fourth predetermined timing, and the fourth power consumption threshold is greater than the third power consumption threshold.

In another embodiment, when it is determined that the release process is not successful in Step S35, the release method further includes:
Step S36: supplying power to the release circuit until the fifth predetermined timing is reached, and determining that the release process is successful at the fifth predetermined timing.

In another preferred embodiment, a reference range of power consumption is also pre-stored according to the release method. It is to be determined whether the stored power consumption is within the reference range of power consumption; if not, it is determined that the release process is terminated, and if so, it is determined that the third release mode is to be executed. This embodiment is an alternative solution provided on the basis that the release process is not successful in both the first release mode and the second release mode, and the release process can be preformed in the third release mode.

In one embodiment, referring to Fig. 6, the process of the third release mode is as follows:
Step S41: acquiring a fourth power consumption, which refers to the power consumption of the release circuit in the time period from the beginning of the third release mode to the sixth predetermined timing;
Step S42: determining whether the fourth power consumption is greater than or equal to the fifth power consumption threshold; if not, it is determined that the first release mode is terminated.

In this embodiment, the third predetermined current value, the sixth predetermined timing, the fifth power consumption threshold and a third predetermined time duration are pre-stored.

In another embodiment, referring to Fig. 6, when it is determined that the fourth power consumption is greater than or equal to the fifth power consumption threshold in Step S42, it is directed to Step S43 and Step S44.
Step S43: acquiring a comparison current, which refers to the current of the release circuit at the timing for comparison; the timing for comparison refers to any timing at which the power consumption of the release circuit in the process of excution of the third release mode is greater than the fifth power consumption threshold;
Step S44: determining whether the comparison current is less than the third predetermined current value; if so, it is directed to Step S45, and if not, it is directed to Step S46;
Step S45: determining whether the the timing for comparison is prior to the sixth predetermined timing; if so, it is directed to Step S46, and if not, it is determined that the second release mode is terminated;
Step S46: acquiring a third accumulated time duration, which refers to the time duration over which the current of the release circuit is less than the third predetermined current value in the time period from the beginning of the third release mode to the sixth predetermined timing;
Step S47: determining whether the third accumulated time duration is greater than or equal to the third predetermined time duration; if so, it is determined that the release process is successful, and if not, it is directed to Step S43.

In another embodiment, the order of Step S44 and Step S45 in Fig. 6 can also be interchanged. In this case, the process of the third release mode is as follows:
Step S41: acquiring a fourth power consumption, which refers to the power consumption of the release circuit in the time period from the beginning of the third release mode to the sixth predetermined timing;
Step S42: determining whether the fourth power consumption is greater than or equal to the fifth power consumption threshold; if so, it is directed to Step S43 and Step S45, and if not, it is determined that the release process is terminated;
Step S43: acquiring a comparison current, which refers to the current of the release circuit at the timing for comparison; the timing for comparison refers to any timing at which the power consumption of the release circuit in the process of excution of the third release mode is greater than the fifth power consumption threshold;
Step S45: determining whether the the timing for comparison is prior to the sixth predetermined timing; if so, it is directed to Step S44, and if not, it is determined that the release process is terminated;
Step S44: determining whether the comparison current is less than the third predetermined current value; if so, it is directed to Step S46;
Step S46: acquiring a third accumulated time duration, which refers to the time duration over which the current of the release circuit is less than the third predetermined current value in the time period from the beginning of the third release mode to the sixth predetermined timing;
Step S47: determining whether the third accumulated time duration is greater than or equal to the third predetermined time duration; if so, it is determined that the release process is successful, and if not, it is directed to Step S43.

The present invention provides at least one of the following beneficial effects:
1) In the general procedure, a detection and a determination on the current environments are performed (in Step S12 as shown in Fig. 3), then different critical current values are set according to different current environments. In different current environments, it is to be determined whether the accumulated time durations for the currents to reach the critical current values reach the predetermined accumulated time durations. As a result, the method distinguishes between high-current environments and low-current environments, and adopts different judgment standards and different judgment steps for different current environments, which can avoid the "one-size-fits-all" judgment standard for detection on release state in the existing method, reduce the misjudgment, and effectively improve the accuracy of judgment.
2) In the general procedure, if the release process performed for the first time is not successful and the power consumption satisfies certain conditions, the release process is automatically performed for the second time (in the third release mode), which effectively improves the user experience.
3) In each release mode, the condition for determining the success of the release process includes the Td index, that is, the accumulated time duration for the current to reach the critical current value, which can effectively reduce the misjudgment caused by the instability of the circuit current and further improve the accuracy.
4) The release circuit in the push rod adopts a self-loop structure, and thus there is no need to insert conductive needles or place electrodes on the human body, which reduces the suffering of the patient.

Although the present invention is disclosed above, it is not limited thereto. Various modifications and variations can be made in the present invention by those skilled in the art without departing from the spirit and scope of the invention. Thus, provided that these modifications and variations of the present invention fall within the scope of the claims of the present invention and their equivalents, the present invention is also intended to include these modifications and variations.

## Claims

1. A release device, applied to a release system, the release device comprising a power supply assembly, a current detector, a first timer and a control unit; wherein,
the power supply assembly is configured to connect with a push rod and supply power to positive and negative electrodes of the push rod; when the power supply assembly supplies power to the positive and negative electrodes of the push rod, the release system forms a release circuit;
the current detector is configured to detect a current of the release circuit;
the first timer is configured to detect an accumulated time duration over which the current of the release circuit is less than a predetermined current value; and,
the control unit is in communication with the current detector and the first timer, and configured to acquire a power consumption of the release circuit and determine whether a release process performed by the release system is successful according to the power consumption and the accumulated time duration.

2. The release device according to claim 1, further comprising a second timer that is in communication with the control unit, and configured to detect a release time duration;
wherein the control unit is configured to calculate the power consumption according to the current of the release circuit and the release time duration.

3. The release device according to claim 1, wherein the control unit pre-stores a first predetermined timing and a first power consumption threshold, and the release device comprises a first release mode and a second release mode, wherein the control unit further pre-stores a first predetermined current value for the first release mode and a second predetermined current value for the second release mode, and the second predetermined current value is greater than the first predetermined current value;
wherein the control unit is further configured to acquire an initial power consumption that refers to a power consumption of the release circuit during a time period from a beginning of the release process to the first predetermined timing, and determine that the first release mode or the second release mode is to be excuted for the release process according to the initial power consumption and the first power consumption threshold.

4. The release device according to claim 3, when the initial power consumption is less than the first power consumption threshold, the control unit is configured to execute the first release mode; the control unit further pre-stores a second predetermined timing and a third predetermined timing both for the first release mode, and the third predetermined timing is after the second predetermined timing;
during a time period from the beginning of the release process to the third predetermined timing, the first timer is configured to acquire a first accumulated time duration that refers to an accumulated time duration over which the current of the release circuit is less than the first predetermined current value in the a time period from the beginning of the release process to the third predetermined timing;
the control unit is configured to acquire a first power consumption that refers to a power consumption of the release circuit in a time period from the beginning of the release process to the second predetermined timing, and determine whether the release process is successful according to the first power consumption and the first accumulated time duration.

5. The release device according to claim 4, wherein the control unit pre-stores a second power consumption threshold and a first predetermined time duration, wherein the power consumption of the release circuit in the time period from beginning of the release process to the third predetermined timing is a second power consumption;
when the control unit determines whether the release process is successful, the control unit is configured to determine whether the first power consumption is less than the second power consumption threshold,
if so, acquire and store the second power consumption, and control the power supply assembly to stop supplying power to the release circuit to suspend the release process, and
if not, determine whether the first accumulated time duration is greater than or equal to the first predetermined time duration,
if not, acquire and store the second power consumption, and control the power supply assembly to stop supplying power to the release circuit to suspend the release process, and
if so, determine that the release process is successful.

6. The release device according to claim 3, wherein when the initial power consumption is greater than or equal to the first power consumption threshold, the control unit is configured to perform the release process in the second release mode;
the control unit pre-stores a fourth predetermined timing for the second release mode, and in a time period from the beginning of the release process to the fourth predetermined timing, the first timer is configured to acquire a second accumulated time duration that refers to an accumulated time duration over which the current of the release circuit is less than the second predetermined current value in the time period from the beginning of the release process to the fourth predetermined timing;
the control unit is configured to acquire a third power consumption that refers to the power consumption of the release circuit in the time period from the beginning of the release process to the fourth predetermined timing, and determine whether the release process is successful according to the third power consumption and the second accumulated time duration.

7. The release device according to claim 6, wherein the control unit pre-stores a third power consumption threshold and a second predetermined time duration;
when the control unit determines whether the release process is successful, the control unit is configured to determine whether the third power consumption is less than the third power consumption threshold,
if not, store the third power consumption, and control the power supply assembly to stop supplying power to the release circuit to suspend the release process, and
if so, determine whether the second accumulated time duration is greater than or equal to the second predetermined time duration,
if so, determine that the release process is successful, and
if not, store the third power consumption, and control the power supply assembly to stop supplying power to the release circuit to suspend the release process.

8. The release device according to claim 7, wherein the control unit further pre-stores a fifth predetermined timing and a fourth power consumption threshold, the fifth predetermined timing is after the fourth predetermined timing, and the fourth power consumption threshold is greater than the third power consumption threshold;
when the second accumulated time duration is greater than or equal to the second predetermined time duration, the control unit is configured to determine whether the third power consumption is greater than or equal to the fourth power consumption threshold, if so, determine that the release process is successful, and if not, control the power supply assembly to continue supplying power to the release circuit until the fifth predetermined timing is reached, and determine that the release process is successful at the fifth predetermined timing.

9. The release device according to claim 5 or 7, further comprising a third release mode, and the control unit further pre-stores a reference range of power consumption;
after the release process is suspended, the control device is further configured to determine whether the stored power consumption is within the reference range of power consumption, if yes, execute the third release mode, and if not, determine that the release process is terminated.

10. The release device according to claim 9, wherein the control unit pre-stores a third predetermined current value, a sixth predetermined timing and a fifth power consumption threshold;
in the third release mode, the control unit is configured to: acquire a fourth power consumption that refers to the power consumption of the release circuit in a time period from the beginning of the third release mode to the sixth predetermined timing; determine whether the fourth power consumption is greater than or equal to the fifth power consumption threshold; determine whether a comparison current is less than the third predetermined current value, the comparison current refers to the current of the release circuit at a timing for comparison, the timing for comparison refers to a timing when the power consumption of the release circuit in the third release mode is greater than the fifth power consumption threshold; determine whether the timing for comparison is before the sixth predetermined timing; determine whether the third accumulated time duration is greater than or equal to the third predetermined time duration, the third accumulated time duration is an accumulated time duration over which the current of the release circuit is less than the third predetermined current value in the time period from the beginning of the third release mode to the sixth predetermined timing, if the third accumulated time duration is greater than or equal to the third predetermined time duration, it is determined that the release process is successful.

11. The release device according to claim 10, wherein, in the third release mode, the control unit is further configured to determine that the release process is terminated when the fourth power consumption is less than the fifth power consumption threshold, or when the comparison current is greater than the third predetermined current value.

12. The release device according to claim 1, further comprising a display element that is in communication with the control unit, and configured to display a release state of the release system.

13. The release device according to claim 1, wherein the power supply assembly comprises a constant current source and a current driver, wherein the constant current source and the current driver are both connected to the control unit, and the constant current source is configured to supply power to the release circuit through the current driver.

14. A release system, comprising a push rod and the release device according to any one of claims 1-13, wherein the push rod is configured to connect with the power supply assembly of the release device, and the power supply assembly is configured to supply power to positive and negative electrodes of the push rod to form the release circuit.

15. The release system according to claim 14, wherein the power supply assembly has a positive electrode and a negative electrode, and the push rod comprises a first electrical conductor and a second electrical conductor, wherein the first electrical conductor is connected to the negative electrode of the power supply assembly, the second electrical conductor comprises a conductive area and a release area that are connected to each other, wherein the conductive area is connected to the positive electrode of the power supply assembly, and the release area is configured to connect with the first electrical conductor through an electrolyte, wherein the power supply assembly is configured to supply power to the push rod to break the release area.

16. A therapeutic device, comprising a medical implant and the release system according to claim 14 or 15, the medical implant is configurd to connect with the release system, and can be release from the release system in a predetermined condition.

17. A release method, comprising steps of:
acquiring the power consumption of a release circuit;
acquiring an accumulated time duration over which a current of the release circuit is less than a predetermined current value; and
determining whether a release process performed by a release system is successful according to the power consumption and the accumulated time duration.

18. The release method according to claim 17, wherein the release process performed by the release system is determined to be successful when the power consumption reaches a predetermined power consumption threshold, and when the accumulated time duration reaches a predetermined time duration.

19. The release method according to claim 18, comprising:
determining in advance whether the power consumption reaches the predetermined power consumption threshold, and if so, determining whether the accumulated time duration reaches the predetermined time duration.

20. The release method according to claim 17, wherein the predetermined current value is set according to a detection result of the present power consumption of the release circuit;
when the power consumption is less than the first power consumption threshold, a first predetermined current value is set;
when the power consumption is greater than or equal to the first power consumption threshold, a second predetermined current value is set;
wherein the first predetermined current value is less than the second predetermined current value.

21. The release method according to claim 20, comprising a first release mode and a second release mode;
wherein the release method comprises:
Step S1: determining whether the first release mode or the second release mode is to be selected for the release process to be performed for a first time according to whether the present power consumption of the release circuit reaches the first power consumption threshold;
Step S2: performing the release process for the first time, and determining that the release process performed for the first time is successful and the release process is terminated when the power consumption reaches a predetermined power consumption threshold and when the accumulated time duration reaches a predetermined time duration.

22. The release method according to claim 21, further comprising a third release mode;
when the release process is determined to be not successful in Step S2, the release method further comprises:
Step S3: determining whether the third release mode is to be excuted according to whether the power consumption of the release circuit reaches a preset range; if not, it is determinated that the release process is terminated, and if so, it is directed to Step S4;
Step S4: executing the third release mode to perform the release process for a second time; and determining that the release process performed for the second time is successful and the release process is terminated when the power consumption reaches the predetermined power consumption threshold and when the accumulated time duration reaches the predetermined time duration.

23. The release method according to claim 21, wherein, in Step S1, the first release mode or the second release mode is selected for the release process to be performed for the first time according to:
Step S 11: acquiring an initial power consumption that refers to a power consumption of the release circuit during a time period from a beginning of the release process to a first predetermined timing;
Step S12: determining whether the initial power consumption is less than the first power consumption threshold; if so, it is determined that the first release mode is to be selected for the release process; if not, it is detertmined that the second release mode is to be selected for the release process.

24. The release method according to claim 22, wherein the first predetermined current value, a second predetermined timing, a third predetermined timing, a second power consumption threshold and a first predetermined time duration are pre-stored for the first release mode, wherein the third predetermined timing is after the second predetermined timing;
wherein the first release mode is excuted through:
Step S21: acquiring a first power consumption that refers to the power consumption of the release circuit during a time period from the beginning of the release process to the second predetermined timing;
Step S22: determining whether the first power consumption is greater than or equal to the second power consumption threshold; if so, it is directed to Step S23 and Step S24; if not, it is directed to Step S25;
Step S23: acquiring a first accumulated time duration that refers to the accumulated time duration over which the current of the release circuit is less than the first predetermined current value during a time period from the beginning of release process to the third predetermined timing;
Step S24: determining whether the first accumulated time duration is greater than or equal to the first predetermined time duration; if so, it is determined that the release process is successful.

25. The release method according to claim 24, wherein,
when the release process is determined to be not successful in Step S24, the release method further comprises:
Step S25: acquiring and storing a second power consumption that refers to the power consumption of the release circuit in the time period from the beginning of the release process to the third predetermined timing, and stopping supplying power to the release circuit to suspend the release process.

26. The release method according to claim 22, wherein the second predetermined current value, a fourth predetermined timing, a fifth predetermined timing, a third power consumption threshold, a fourth power consumption threshold, and a second predetermined time duration are pre-stored for the second release mode, wherein the fifth predetermined timing is after the fourth predetermined timing, and the fourth power consumption threshold is greater than the third power consumption threshold;
the second release mode is excuted through:
Step S31: acquiring a third power consumption that refers to the power consumption of the release circuit in a time period from the beginning of the release process to the fourth predetermined timing;
Step S32: determining whether the third power consumption is greater than or equal to the third power consumption threshold; if so, it is directed to Step S33 and Step S34, and if not, it is directed to Step S37;
Step S33: acquiring a second accumulated time duration that refers to a time duration over which the current of the release circuit is less than the second predetermined current value during the time period from the beginning of release process to the fourth predetermined timing;
Step S34: determining whether the second accumulated time duration is greater than or equal to the second predetermined time duration; if so, it is directed to Step S35, and if not, it is directed to Step S37;
Step S3 5: determining whether the third power comsuption is greater than or equal to the fourth power consumption threshold; if so, it is determined that the release process is successful;
Step S37: storing the third power consumption, and stopping supplying power to the release circuit to suspend the release process.

27. The release method according to claim 26, wherein,
when it is determined that the release process is not successful in Step S35, the release method further comprises:
Step S36: supplying power to the release circuit until the fifth predetermined timing is reached, and determining that the release process is successful at the fifth predetermined timing.

28. The release method according to any one of claims 25-27, wherein
it is to be determined whether the stored power consumption is within a reference range of power consumption; if not, it is determined that the release process is terminated, and if so, it is determined that the third release mode is to be excuted.

29. The release method according to claim 22, wherein a third predetermined current value, a sixth predetermined timing, a fifth power consumption threshold and a third predetermined time duration are pre-stored for the third release mode;
the third release mode is excuted through:
Step S41: acquiring a fourth power consumption that refers to the power consumption of the release circuit in a time period from a beginning of the third release mode to the sixth predetermined timing;
Step S42: determining whether the fourth power consumption is greater than or equal to the fifth power consumption threshold; if not, it is determined that the first release mode is terminated.

30. The release method according to claim 29, wherein, when it is determined that the fourth power consumption is greater than or equal to the fifth power consumption threshold in Step S42, it is directed to Step S43 and Step S44.
Step S43: acquiring a comparison current that refers to the current of the release circuit at the timing for comparison; the timing for comparison is a timing at which the power consumption of the release circuit in the third release mode is greater than the fifth power consumption threshold;
Step S44: determining whether the comparison current is less than the third predetermined current value; if so, it is directed to Step S45, and if not, it is directed to Step S46;
Step S45: determining whether the the timing for comparison is prior to the sixth predetermined timing; if so, it is directed to Step S46, and if not, it is determined that the second release mode is terminated;
Step S46: acquiring a third accumulated time duration that refers to a time duration over which the current of the release circuit is less than the third predetermined current value during the time period from the beginning of the third release mode to the sixth predetermined timing;
Step S47: determining whether the third accumulated time duration is greater than or equal to the third predetermined time duration; if so, it is determined that the release process is successful, and if not, it is directed to Step S43.
